# EUROPEAN PATENT SPECIFICATION

(11) **EP 3 765 153 B1**
(45) Date of publication and mention of the grant of the patent: **29.03.2023**
(21) Application number: 19714917.2
(22) Date of filing: 13.03.2019
(51) Int. Cl.: A61N 5/10, G21K 1/04, A61B 6/06, A61B 6/00

(54) **LIMITING IMAGING RADIATION DOSE AND IMPROVING IMAGE QUALITY**
BEGRENZUNG DER ABBILDUNGSSTRAHLUNGSDOSIS UND VERBESSERUNG DER BILDQUALITÄT
LIMITATION DE LA DOSE DE RAYONNEMENT D'IMAGERIE ET AMÉLIORATION DE LA QUALITÉ D'IMAGE

(30) Priority: 15.03.2018 US 201815922688
(43) Date of publication of application: 20.01.2021
(73) Proprietor: Accuray Incorporated, Sunnyvale, CA 94089 (US)
(72) Inventor: BENEKE, Matthew, Madison, Wisconsin 53705 (US); JORDAN, Petr, Redwood City, California 94062 (US); LAING, Trevor, San Jose, California 95125 (US); MAURER, Calvin R. Jr., San Jose, California 95126 (US)
(74) Representative: McKinnon, Alistair James
(86) International application number: PCT/US2019/022109
(87) International publication number: WO 2019/178270

(56) References cited:
- US-A1- 2004 202 283
- US-A1- 2009 168 966

## Description

### TECHNICAL FIELD

Embodiments of the disclosure relate to the field of radiation treatment delivery imaging.

### BACKGROUND

The intra-fraction motion of tumors during radiation therapy treatments is a concern in the modern era of image-guided radiotherapy. Providers of radiation oncology treatment systems have incorporated a kV x-ray imaging systems to obtain 2D radiographs of the radiation target, providing information about tumor position in real-time. This information can then be used by the treatment system to modify the delivery of therapeutic dose and compensate for the tumor motion.

In parallel to these developments, the radiation dose absorbed by patients from medical imaging procedures has come under scrutiny. The use of x-ray imaging system in radiation treatment procedures adds to the radiation dose absorbed by patients.

Documents US2009168966 A1 and US2004202283 A1 both use a variable collimator to control the area imaged.

### SUMMARY OF EMBODIMENTS

In one embodiment, an apparatus comprises an x-ray imaging source of a helical delivery system, wherein the x-ray imaging source comprises a variable aperture collimator. The collimator may comprise a multi-leaf collimator. Alternatively, the collimator may comprise an iris collimator.

The claimed invention is defined in the claims, and further embodiments in the dependent claims.

### BRIEF DESCRIPTION OF THE DRAWINGS

Embodiments of the present disclosure are illustrated by way of example, and not by way of limitation, in the figures of the accompanying drawings.
**Figure 1** illustrates a helical delivery system in accordance with embodiments of the present disclosure.
**Figure 2** is a cross-section of the treatment imaging system of **Figure 1****.**
**Figure 3** is a flowchart illustrating a method for adjusting the aperture of a collimator of a kV imaging source to image a region of interest in accordance with embodiments of the present disclosure.
**Figure 4** is an illustration of generating an x-ray image having a full field of view (FOV) from a first angle.
**Figure 5** is an illustration of generating an x-ray image having a full FOV from a second angle.
**Figure 6A** is an illustration of the identification of a region of interest (ROI) in the x-ray image generated in **Figure 4****.**
**Figure 6B** is an illustration of the identification of a ROI in the x-ray image generated in **Figure 5****.**
**Figure 7** is an illustration of the collimator of **Figure 1** according to embodiments of the present disclosure.
**Figure 8** is an illustration of generating an x-ray image having a partial FOV from the first angle.
**Figure 9** is an illustration of generating an x-ray image having a partial FOV from the second angle.
**Figure 10** is an illustration of a method of adjusting the aperture of the collimator of the kV imaging source while the kV imaging source rotates from the first angle to the second angle.
**Figure 11A** is an illustration of the x-ray image from the first angle generated in **Figure 8****.**
**Figure 11B** is an illustration of the x-ray image from the second angle generated in **Figure 9****.**
**Figure 12** illustrates the configuration of an image-guided radiation treatment system in which embodiments of the present disclosure may be practiced.
**Figure 13** illustrates the configuration of gantry based radiotherapy system in which embodiments of the present disclosure may be practiced.
**Figure 14** illustrates the configuration of a portal imaging system in which embodiments of the present disclosure may be practiced.

### DETAILED DESCRIPTION

In order to control a radiation therapy procedure, said control not forming part of any claimed method, the location of a target region (e.g., tumor) that is to be treated may be tracked with the use of a kilovoltage (kV) x-ray imaging system. This can be particularly important for targets that have the potential to move during treatment, such as target regions on or near a lung, on or near a heart, on or near a prostate, and so on. For some types of target regions, existing techniques of tracking the target regions may not be optimal due to exposure of a patient to additional radiation. For example, some target regions may require an increase in the energy of the imaging x-ray beam in order to get a useable x-ray image which exposes the patient to increased amounts of radiation. In another example, to accurately treat a target region that is moving within a patient, x-ray images of the target region may be generated at a sufficient frequency to verify the location of the target region, exposing the patient to additional radiation. Embodiments of the present disclosure relate to methods and systems for generating and using images of a smaller region of interest (ROI) within a treatment volume of interest (VOI) of a patient during radiation treatment delivery to minimize the patient's exposure to additional radiation.

During treatment delivery, dynamic tracking of the ROI may be performed based on the use of x-ray images taken to identify a target region. Once the location of the target region has been computed, the treatment delivery (e.g., radiation beam source position of the radiation treatment delivery system) may be adjusted to compensate for the dynamic motion of the target. In order to accurately track the motion of the target region, an x-ray imaging system may be used to continuously generate x-ray images of the target within a patient throughout the treatment. However, a result may be exposure of the patient to additional radiation.

An embodiment of the present disclosure may minimize the exposure to additional radiation issue described above by adjusting the field of view (FOV) of the kV imaging source with a collimator to a smaller field of view for a more specific region of interest (ROI) around the treatment target. Based on an identification of the ROI in, for example, a full FOV of the x-ray imaging system, an aperture of a collimator of the kV imaging source may be adjusted to correspond to the size or shape of the ROI, resulting in x-ray images of the ROI that may have a smaller FOV than the full FOV x-ray images generated during, for example, a patient set up stage. This may allow for the generation of x-ray images of the ROI having the same, or better, quality as the x-ray images with the full FOV without exposing the healthy tissue surrounding the ROI to unwanted radiation and decreasing the overall image radiation dose to the patient. In other words, embodiments of the present disclosure may reduce the amount of tissue being irradiate and, thereby, reduce the scatter and thus improve the image quality of the reduced FOV being imaged versus the image quality of that region with a larger FOV (e.g., uncollimated).

It should be noted that in some embodiments described below, but not in any claimed method, the same radiation source may be used to provide both higher energy therapeutic radiation and lower energy imaging radiation. In such an embodiment, the radiation source may be a linear acceleration (LINAC) having a primary collimator and a secondary collimator. The primary collimator may have a fixed aperture and is positioned after the electron beam has passed through the x-ray emitting target. The secondary collimator may be positioned after the primary collimator and may have a variable aperture, as will be discussed in more detail below.

Embodiments of the disclosure are particularly applicable to a radiosurgical treatment system and method and it is in this context that these embodiments of the disclosure will be described. It will be appreciated, however, that the system and method in accordance with the disclosure has greater utility, such as to other types of treatments wherein it is necessary to accurately position treatment at a target region within the patient in order to avoid damaging healthy tissue such as to other types of medical procedures with other types of medical instruments.

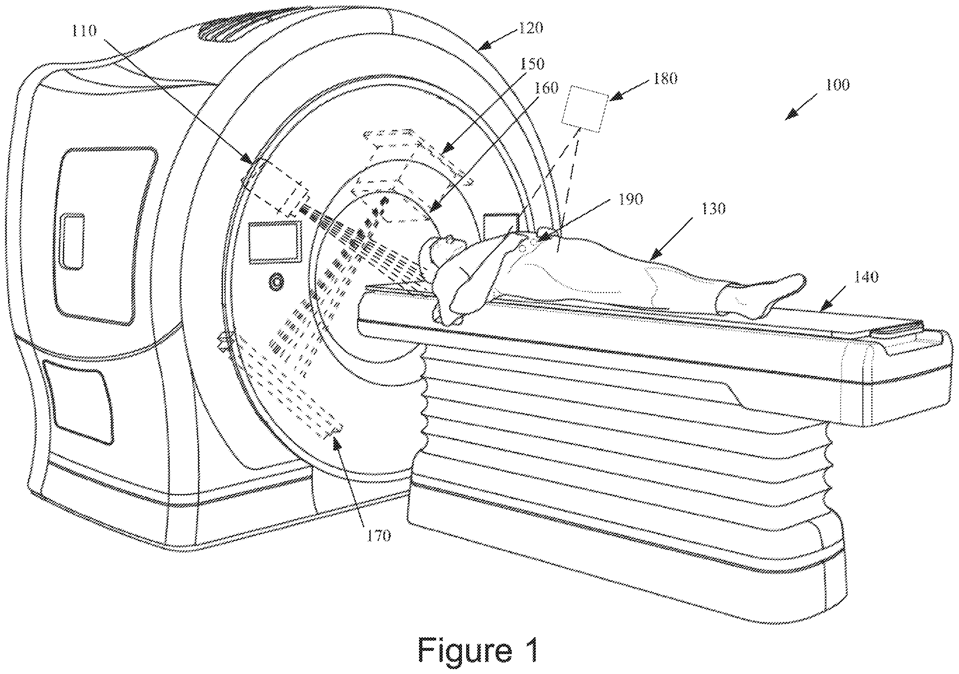

**Figure** 1 illustrates a helical delivery system 100 in accordance with embodiments of the present disclosure. The helical delivery system 100 of **Figure** 1 may include a linear accelerator (LINAC) 110 mounted to a ring gantry 120. The LINAC 110 may be used to generate a narrow intensity modulated pencil beam (i.e., treatment beam) by directing an electron beam towards an x-ray emitting target. The treatment beam may deliver radiation to a target region (i.e., a tumor). The ring gantry 120 generally has a toroidal shape in which the patient 130 extends through a bore of the ring/toroid and the LINAC 110 is mounted on the perimeter of the ring and rotates about the axis passing through the center to irradiate a target region with beams delivered from one or more angles around the patient. During treatment, the patient 130 may be simultaneously moved through the bore of the gantry on treatment couch 140.

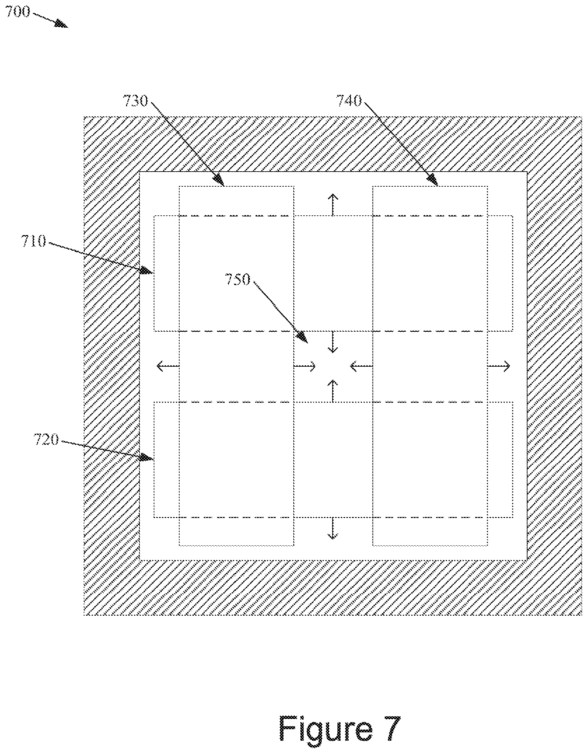

The helical delivery system 100 of **Figure 1** includes a treatment imaging system, which may include a kV imaging source 150 and an x-ray detector 170. The kV imaging source 150 may be used to generate x-ray images of a ROI of patient 130 by directing a sequence of x-ray beams at the ROI which are incident on the x-ray detector 170 opposite the kV imaging source 150 to image the patient 130 for setup and generate in-treatment images. The treatment imaging system may further include a collimator 160. In one embodiment, the collimator 160 may be a variable aperture collimator as discussed in **Figure 7****.** In another embodiment, the collimator 160 may be a multi-leaf collimator (MLC). The MLC includes a housing that houses multiple leaves that are movable to adjust an aperture of the MLC to enable shaping of an imaging x-ray beam. In another embodiment, the variable aperture collimator 160 may be an iris collimator containing trapezoidal blocks that move along a frame in a manner similar to a camera iris to produce an aperture of variable size that enables shaping of the imaging x-ray beam. The kV imaging source 150 and the x-ray detector 170 may be mounted orthogonally relative to the LINAC 110 (e.g., separated by 90 degrees) on the ring gantry 120 and may be aligned to project an imaging x-ray beam at a target region and to illuminate an imaging plane of detector 170 after passing through the patient 130. In some embodiments, the LINAC 110 and/or the kV imaging source 150 may be mounted to a C-arm gantry in a cantilever-like manner, which rotates the LINAC 110 and kV imaging source 150 about the axis passing through the isocenter.

In one embodiment, the radiation therapy system 100 may include a motion detection device 180 to determine target motion. The motion detecting device 180 may detect external patient motion (such as chest movement during respiration) that occurs within an imaging field. The motion detecting device 180 can be any sensor or other device capable of identifying target movement. The motion detecting device 180 may be, for example, an optical sensor such as a camera, a pressure sensor, an electromagnetic sensor, or some other sensor that can provide motion detection without delivering ionizing radiation to a patient 130 (e.g., a sensor other than an x-ray imaging system). In one embodiment, the motion detecting device 180 acquires measurement data indicative of target motion in real-time. Alternatively, the measurement data may be acquired at a frequency that is higher (potentially substantially higher) than can be achieved or than is desirable with x-ray imaging (due to ionizing radiation delivered to the patient 130 with each x-ray image). In one embodiment, the motion detecting device 180 does not provide a high absolute position accuracy. Instead, the motion detecting device 180 may provide sufficient relative position accuracy to detect patient movement and/or target movement.

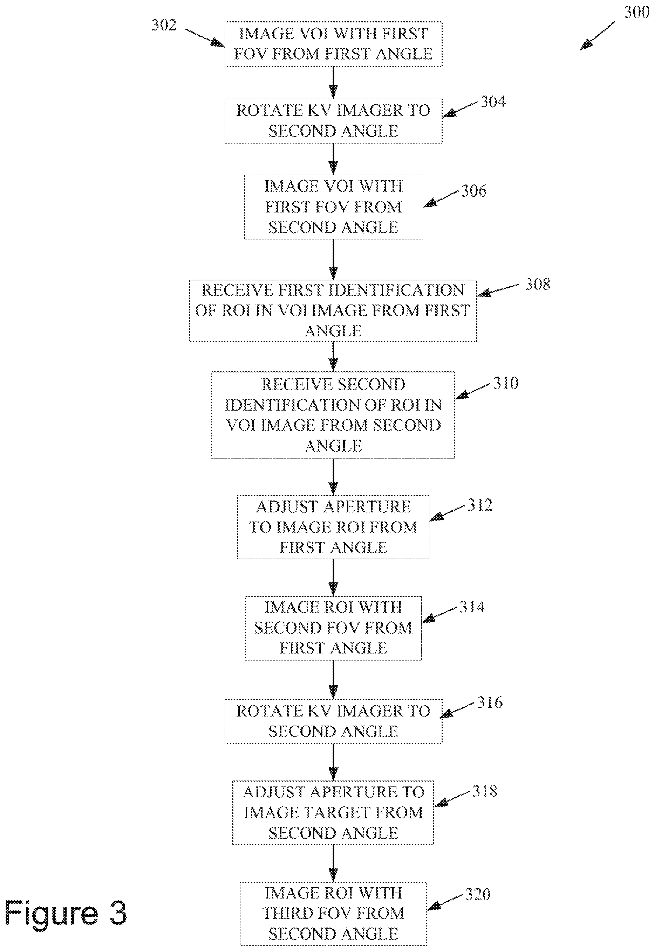

In one embodiment, the motion detecting device 180 is an optical system, such as a camera. The optical system may track the position of light-emitting diodes (LEDs) 190 situated on patient 130. Alternatively, the optical system may directly track a surface region (e.g., skin surface) of patient 130, as distinguished from tracking LEDs 190 on the patient 130. There may be a correlation between movement of a target region, described in more detail at **Figures 3-6** below, and movement of the LEDs and/or surface region of the patient 130. A correlation model may be generated between the position of the target region and the tracking LEDs 190 and/or surface region of the patient 130 to predict the position of the target region at a later time. Based on the prediction, the aperture of the collimator 160 of the kV imaging source 150 may be adjusted to correspond to the predicted position of the target region. Adjustment of the aperture of the collimator 160 will be described in more detail at **Figure 7** below.

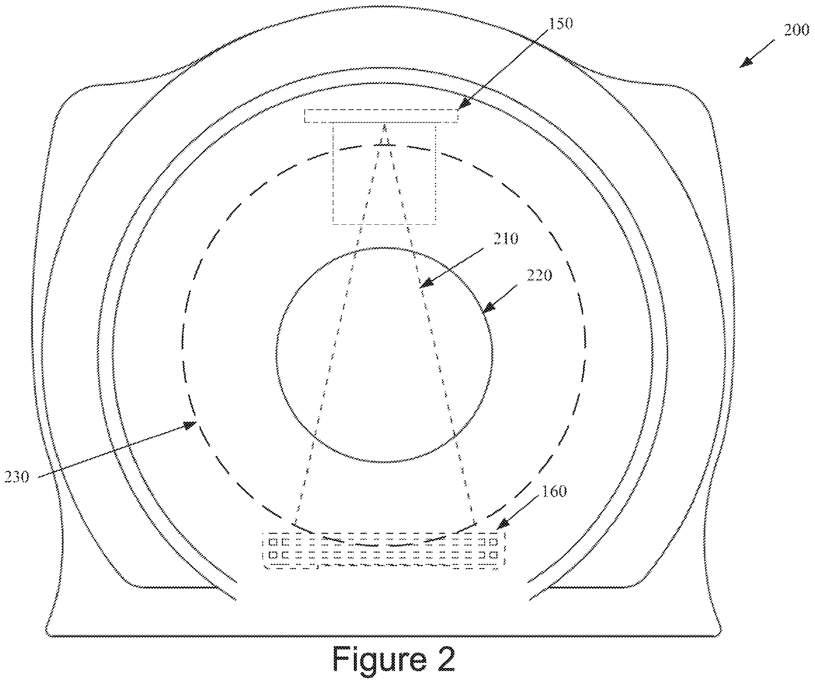

**Figure 2** is a cross-section 200 of the treatment imaging system of **Figure 1****.** As previously discussed, the kV imaging source 150 projects an imaging x-ray beam 210 through the bore 220 of the treatment system which illuminates the imaging plane of x-ray detector 170 after passing through a patient. The kV imaging source 150 and x-ray detector 170 may rotate along a circular track 230 of the ring gantry 120 around the bore 220 of the treatment system to generate x-ray images of a target region from multiple angles.

**Figure 3** is a flowchart 300 illustrating a method of adjusting the aperture of a collimator of a kV imaging source to image a target region in accordance with embodiments of the present disclosure. The method of **Figure 3** allows for the identification of a ROI including a target region from a full FOV image of a VOI. Using the identification, the treatment imaging system may adjust the aperture of the collimator 160 to correspond to the identified ROI, minimizing radiation exposure of tissue surrounding the ROI. At block 302, a kV imaging source 150 and an x-ray detector 170 generate an image of a VOI of a patient that contains a target region from a first angle. At block 304, after generating an x-ray image of the VOI of the patient from the first angle, the kV imaging source 150 and x-ray detector 170 may rotate to a second angle. At block 306, the kV imaging source 150 and x-ray detector 170 generate an image of the VOI of the patient that contains the target region from a second angle. In some embodiments, the first angle and second angle may be determined prior to treatment using a treatment planning system. The images of the VOI may be captured with a full FOV where the collimation device may not shape the imaging x-ray beam. In some embodiments, an ROI including the target region may be identified during a treatment planning phase and images of the VOI may be captured with a partial FOV corresponding to the ROI identified during treatment planning.

At block 308, the treatment imaging system receives an identification of a ROI that includes the target region in the VOI image generated at block 302. In some embodiments, the target region may be identified by a user selecting the target region from the generated image. In other embodiments, the target region may be identified by the treatment imaging system using image recognition software or the like. At block 310, the treatment imaging system receives an identification of a ROI that includes the target region in the VOI image generated at block 306. The target region may be identified using methods similar to those described at block 308. At block 312, the aperture of the collimator 160 of the kV imaging source 150 may be adjusted using the methods previously described to correspond to the ROI identified at block 308. The adjustment of the aperture may allow the kV imaging source 150 and x-ray detector 170 to generate images with a partial FOV that is smaller than the FOV at block 302. At block 314, the kV imaging source 150 and x-ray detector 170 generate an image with the partial FOV of the ROI from the first angle, where the resultant image may correspond to the ROI identified at block 308. At block 316, after generating an x-ray image of the ROI of the patient from the first angle, the kV imaging source 150 and x-ray detector 170 may rotate to the second angle. At block 318, the aperture of the collimator 160 of the kV imaging source 150 may be adjusted using the methods previously described to correspond to the ROI identified at block 310. The adjustment of the aperture may allow the kV imaging source 150 and x-ray detector 170 to generate images with a partial FOV that is smaller than the FOV at block 306. At block 320, the kV imaging source 150 and x-ray detector 170 generate an image with the partial FOV of the ROI from the second angle, where the resultant image may correspond to the ROI identified at block 310. The present embodiment describes imaging an ROI with a partial FOV to reduce the radiation dose delivered to patient 130. However, the actual tracking of a target region may be used in conjunction with other techniques using external markers attached to the patient 130 to correlate movement of the external markers to movement of the target region. An example of one such system is the Synchrony^{™} respiratory tracking system developed by Accuray, Inc.

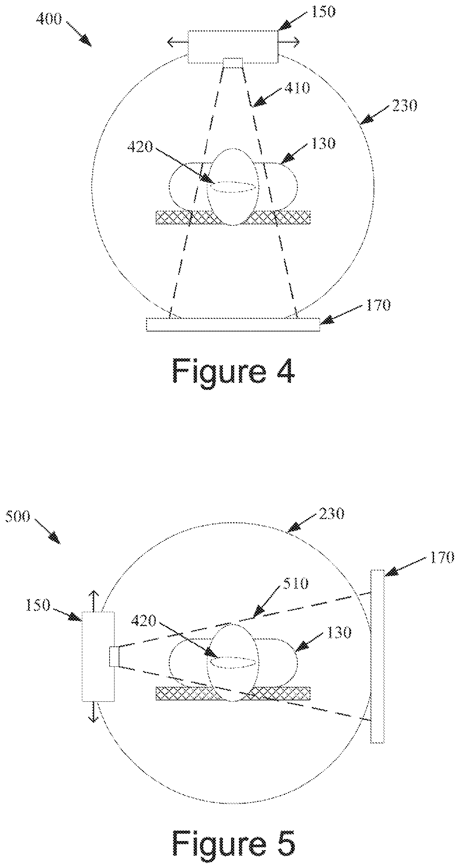

**Figure 4** is an illustration 400 of generating an x-ray image having a full FOV from the first angle. The illustration shown in **Figure 4** may be representative of block 302 of the method of **Figure 3** according to embodiments of the present disclosure. The kV imaging source 150 and the x-ray detector 170 generate an imaging x-ray beam 410 having a full FOV of a VOI of the patient 130 that contains a target region 420 from a first angle. The imaging x-ray beam 410 passes through the patient 130 and the target region 420 and is incident on the x-ray detector 170. The imaging x-ray beam 410 illuminates an imaging plane of the x-ray detector 170 after passing through the patient 130, which may be used by the treatment imaging system to generate an x-ray image of the VOI containing the target region 420 at a first position from the first angle. Once the x-ray image of the VOI from the first angle is acquired, the kV imaging source 150 and the x-ray detector 170 may rotate along the circular track 230 to a second angle as previously discussed at block 304. The present embodiment excludes therapeutic methods in which the movement of the patient is tracked and where a treatment step is performed after imagining of the VOI at the first angle but before imaging of the VOI at the second angle.

Once the kV imaging source 150 and the x-ray detector 170 arrive at the second angle position, an image covering the full FOV of the VOI may be generated. The illustration 500 shown in Figure 5 may be representative of block 306 of the method of Figure 3 according to embodiments of the present disclosure. The kV imaging source 150 and the x-ray detector 170 generate an imaging x-ray beam 510 having a full FOV of a VOI of the patient 130 that contains a target region 420 from a second angle. The imaging x-ray beam 510 passes through the patient 130, which may be used by the treatment imaging system to generate an x-ray image of the VOI containing the target region 420 from the second angle.

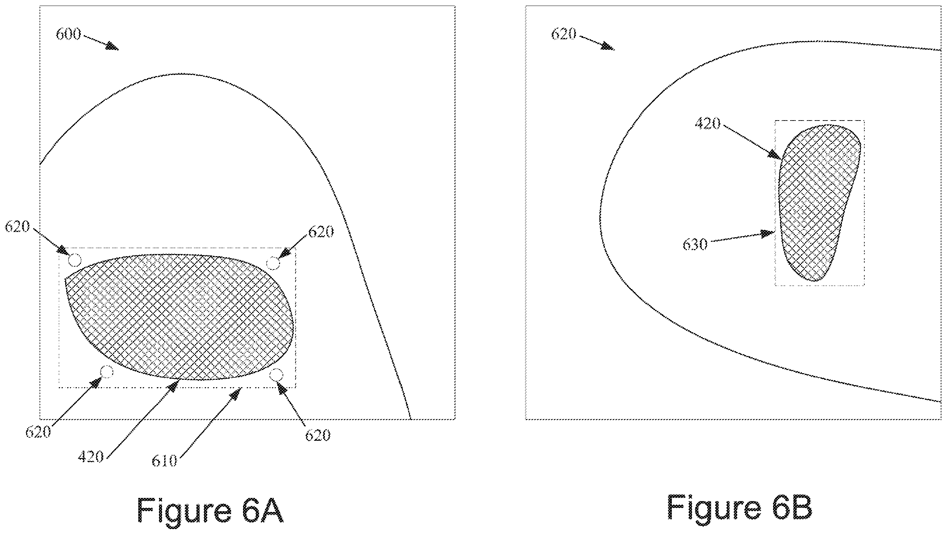

**Figure 6A** is an illustration of the identification of a region of interest (ROI) in the x-ray image generated in **Figures 4****.** The generated x-ray image 600 of the VOI from the first angle may include the target region 420 and internal reference structures 620 located near the target region 420. In the illustrated embodiment, the internal reference structures 620 may be fiducial markers that are implanted in or near the target region 420 and that are visible in the x-ray image 600. In another embodiment, the internal reference structure 620 may be a bone of patient 130 located in close proximity to the target region 420. In some embodiments, it may be possible to generate a visible x-ray image of the target region 420 and direct target imaging of the target region 420 may be performed. The treatment system may then receive an identification of a ROI 610 from the first angle that includes the target region 420. As previously described, in some embodiments the identification of the ROI may be based on a selection of the ROI by a user. Selection of the ROI by the user may be completed by the user selecting the ROI from a user interface of the helical delivery system. In other embodiments, the ROI may be identified automatically by the helical delivery system using image recognition software or the like. In other embodiments, selection of the ROI may be received from a treatment planning system or a prediagnostic scan.

**Figure 6B** is an illustration of the identification of a ROI in the x-ray image generated in **Figures 5****.** The generated x-ray image 620 of the VOI from the second angle may contain the target region 420 as well as the VOI surrounding the target region. The treatment system may then receive an identification of a ROI 630 from the second angle that includes the target region 420. Identification of the ROI 630 may be completed using methods similar to the methods described in **Figure 6A****.**

**Figure 7** is an illustration 700 of the collimator 160 of **Figure 1** according to embodiments of the present disclosure. In one embodiment, the collimator 160 may be a variable aperture collimator. The aperture 750 of the collimator 160 of the kV imaging source 150 may be adjusted as previously described at block 312 and block 318 of **Figure 3****.** The size and/or shape of the aperture may be adjusted to correspond to the ROI identified in **Figures 6A** and **6B****.** The collimator 160 may be composed of a series of plates 710, 720, 730, 740, where the positioning of the plates create aperture 750 in the center of the plates. An imaging x-ray beam passes through the aperture 750 and the shape of the imaging x-ray beam may correspond to the shape of the aperture 750. Therefore, the size and shape of the x-ray beam may be adjusted by changing the size and shape of the aperture 750 by moving plates 710, 720, 730, 740 within the collimator 160. The plates 710, 720, 730, 740 may be made of a material that prevents the passage radiation, such as lead. Plates 710 and 720 may be located on a parallel plane within the collimator 160 and may move vertically independent of one another along a vertical axis. Plates 730 and 740 may be located on a parallel plane within the collimator 160 and may move horizontally independent of one another along a horizontal axis. In some embodiments, plates 710, 720, 730, 740 may be moved by a series of actuators coupled to plates 710, 720, 730, 740 that may extend or retract. In another embodiment, plates 710, 720, 730, 740 may be moved manually by a user sliding the plates along the horizontal or vertical axes and securing plates 710, 720, 730, 740 in place with fasteners. Thus, the aperture 750 and the x-ray beam may be adjusted to various rectangular shapes having various sizes. As previously described, in some embodiments the collimator 160 may be a variable aperture collimator containing trapezoidal blocks that move along a frame in a manner similar to a camera iris to produce an aperture of variable size that enables shaping of the imaging x-ray beam.

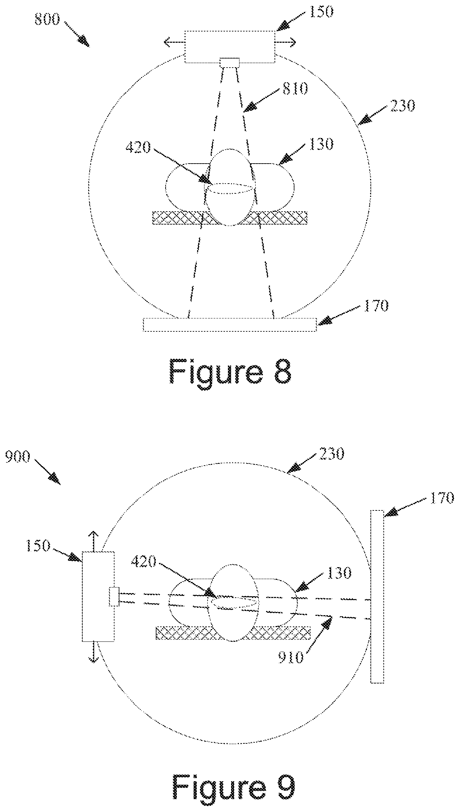

**Figure 8** is an illustration 800 of generating an x-ray image having a partial FOV from the first angle. The illustration shown in **Figure 8** may be representative of block 314 of the method of **Figure 3** according to embodiments of the present disclosure. Prior to imaging, the collimator 160 may adjust the aperture to correspond to the ROI identified in **Figure 6A** using the methods described in **Figure 7**, which may create a partial FOV for the kV imaging source 150. The kV imaging source 150 may then generate an imaging x-ray beam 810 with the partial FOV of the ROI of the patient 130 that includes the target region 420 from the first angle. The imaging x-ray beam 810 passes through the patient 130 and the target region 420 and incident on the x-ray detector 170. The imaging x-ray beam 810 illuminates an imaging plane of the x-ray detector 170 after passing through the patient 130, which may be used by the treatment imaging system to generate an x-ray image of the ROI including the target region 420 from the first angle. Once the x-ray image of the ROI from the first angle is acquired, the kV imaging source 150 and the x-ray detector 170 may rotate along the circular track 230 to a second angle.

Once the kV imaging source 150 and the x-ray detector 170 arrive at the second angle, an image covering the partial FOV of the ROI from the second angle may be generated. The illustration 900 shown in **Figure 9** may be representative of block 320 of the method of **Figure 3** according to embodiments of the present disclosure. Prior to imaging, the collimator 160 may adjust the aperture to correspond to the ROI identified in **Figure 6B** using the methods described in **Figure 7**, which may create a partial FOV for the kV imaging source 150. The kV imaging source 150 and the x-ray detector 170 may then generate an imaging x-ray beam 910 with the partial FOV of the ROI of the patient 130 that includes the target region 420 from the second angle. The imaging x-ray beam 910 passes through the patient 130 and the target region 420 and are collected by the x-ray detector 170. The imaging x-ray beam 810 illuminates an imaging plane of the x-ray detector 170 after passing through the patient 130, which may be used by the treatment imaging system to generate an x-ray image of the ROI containing the target region 420 from the first angle. Once the x-ray image of the ROI from the first angle is acquired, the kV imaging source 150 and the x-ray detector 170 may rotate along the circular track 230 and return to the first angle, where the imaging sequence may be repeated.

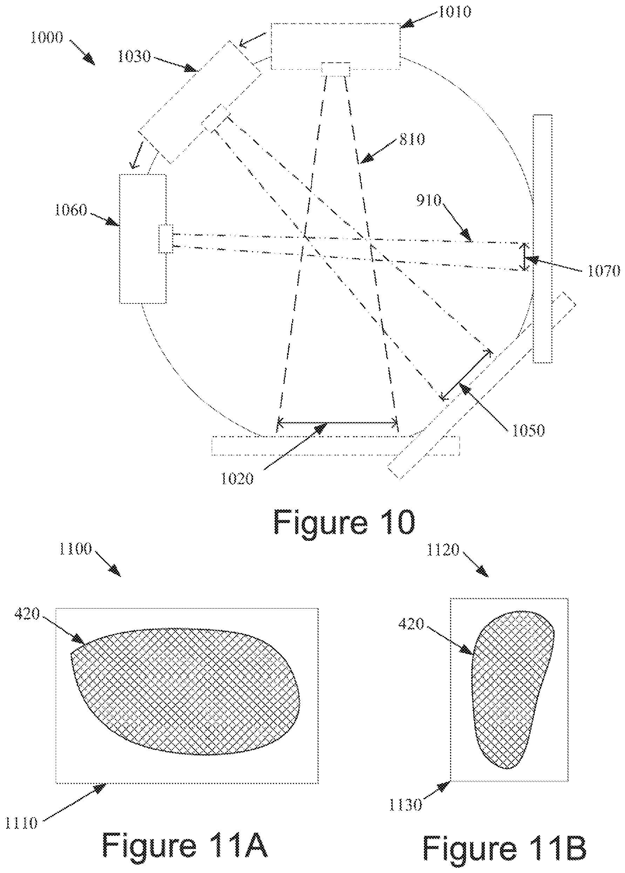

**Figure 10** is an illustration 1000 of a method of adjusting the aperture of the collimator of the kV imaging source 150 while the kV imaging source 150 rotates from the first angle to the second angle. The kV imaging source 150 may be located at the first angle 1010 and may generate an imaging x-ray beam 810 with a FOV 1020 to image the ROI as shown in **Figure 8**. After the x-ray image has been captured, the kV imaging source 150 and x-ray detector 170 may begin to rotate to the second angle. As the kV imager rotates towards the second angle, the aperture of the collimator of the kV imaging source 150 may begin to adjust in accordance with the previously described methods. In one embodiment, at angle 1030 the kV imager may be in the process of rotating between the first angle and the second angle. The aperture of the collimator of the kV imaging source 150 may be adjusting, decreasing the FOV 1050 relative to the FOV 1020 at the first angle 1010. In another embodiment, the aperture of the collimator of the kV imaging source 150 may be adjusted once the kV imager has arrived at the second angle. When the kV imager arrives at the second angle 1060 the adjustment of the aperture of the collimator of the kV imaging source 150 to correspond to the ROI identified in **Figure 6B** may be complete. The kV imager may then generate an imaging x-ray beam 910 with a FOV 1070 to image the ROI as shown in **Figure 9**.

**Figure 11A** is an illustration 1100 of the x-ray image from the first angle generated in **Figure 8**. The x-ray image 1110 may correspond to the ROI identified in **Figure 6A** and include the target region 420 of the patient 130 from the first angle. **Figure 11B** is an illustration 1120 of the x-ray image from the second angle generated in **Figure 9****.** The x-ray image 1130 may correspond to the ROI identified in **Figure 6B** and include the target region 420 of the patient 130 from the second angle. In some embodiments, the imaging sequence may be repeated during a treatment session using the ROI's identified in **Figures 6A** and **6B****.** In other embodiments, the treatment imaging system may receive an identification of a new ROI in the x-ray images of **Figures 11A** and **11B** using the previously described methods and a new imaging sequence may begin.

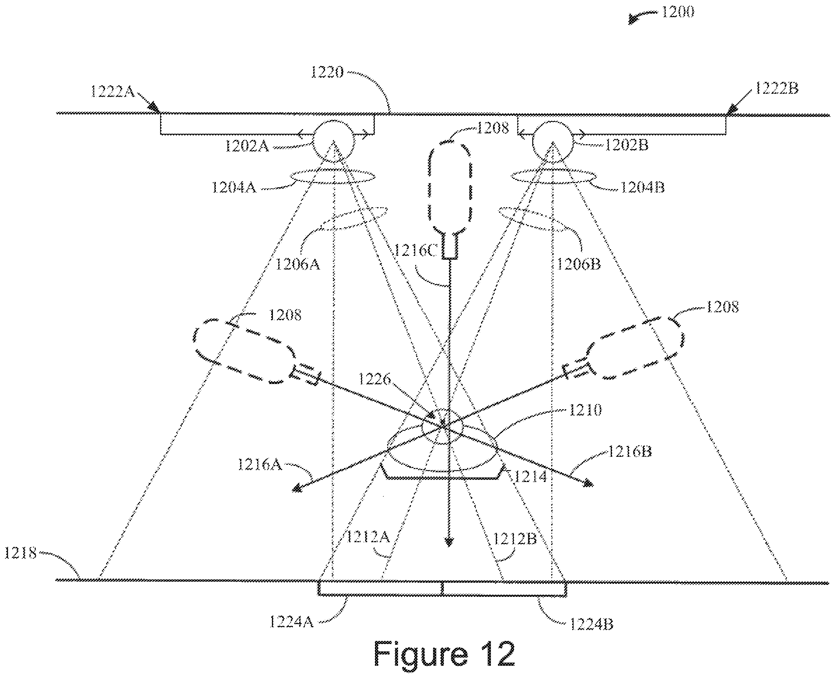

**Figure 12** illustrates the configuration of an image-guided radiation treatment (IGRT) system 1200 in which embodiments of the present disclosure may be practiced. The IGRT system 1200 may include to kV imaging sources 1202A and 1202B and may be mounted on tracks 1222A and 1222B on the ceiling 1220 of an operating room and may be aligned to project imaging x-ray beams 1204 A and 1204B from two different positions such that a ray 1212A of beam 1204 A intersects with a ray 1212B of beam 1204B at an imaging center 1226 (i.e., isocenter), which provides a reference point for positioning the LINAC 1208 to generate treatment beams 1216A, 1216B and 1216C and the patient 1210 on treatment couch 1214 during treatment. After passing through the patient 1210, imaging x-ray beams 1204 A and 1204B may illuminate respective imaging surfaces of x-ray detectors 1224 A and 1224B, which may be mounted at or near the floor 1218 of the operating room and substantially parallel to each other (e.g., within 5 degrees). The kV imaging sources 1202A and 1202B may be substantially coplanar such that the imaging surfaces of kV imaging sources 1202A and 1202B form a single imaging plane. In one embodiment, kV imaging sources 1202 A and 1202B may be replaced with a single kV imaging source. Once an x-ray image of the patient 1214 has been generated, the LINAC 1208 may rotate to generate a treatment beam 1216 from a different angle. While the LINAC 1208 rotates to the different angle, the kV imaging sources 1202A and 1202B may move along tracks 1222 A and 1222B to generate x-ray images of the patient 1210 from a new angle. The present embodiment excludes therapeutic methods in which the movement of the patient is tracked and where a treatment step is performed after imagining of the VOI at the first angle but before imaging of the VOI at the second angle

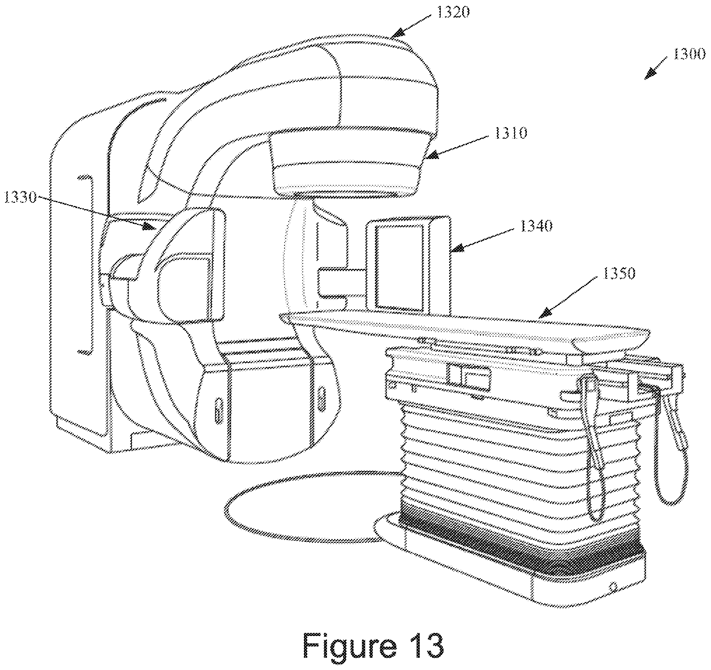

In some embodiments, the kV imaging source and LINAC may be used in other types of gantry-based systems, for example, the TrueBeam^{™} radiotherapy system manufactured by Varian Medical Systems as shown in **Figure 13****.** The radiotherapy system 1300 may include a LINAC 1310 mounted to a C-arm gantry 1320 that rotates about the axis passing through the isocenter. The radiotherapy system may also include a treatment imaging system comprised of a kV imaging source 1330 and an x-ray detector 1340. The kV imaging source 1330 having a variable aperture collimator and the x-ray detector 1340 may be mounted to robotic arms located opposite one another relative to the isocenter of the C-arm gantry 1320 to allow for imaging of a VOI of a patient on a treatment couch 1350.

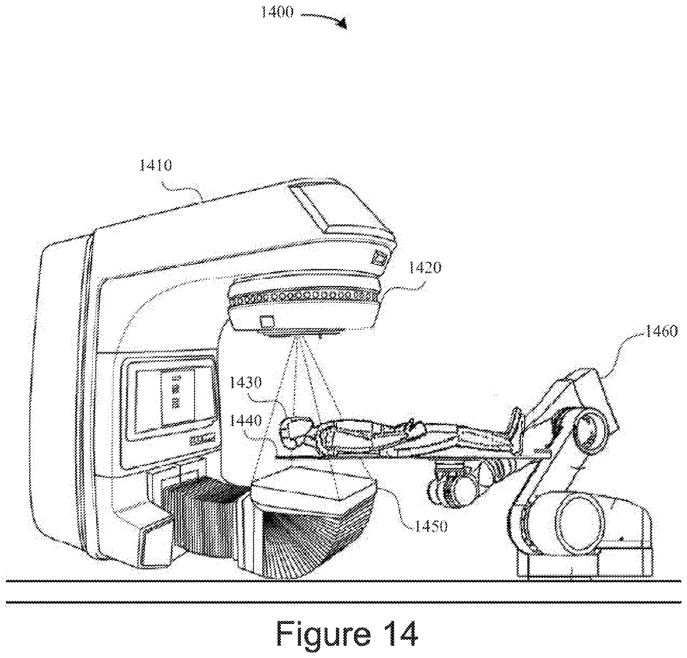

Embodiments of the present disclosure may be implemented in a portal imaging system 1400 as shown in **Figure 14****.** In the portal imaging system 1400 the beam energy of a LINAC may be adjusted during treatment and may allow the LINAC to be used for both x-ray imaging and radiation treatment. The gantry based radiation system 1400 includes a gantry 1410, a radiation source 1420 (i.e. a LINAC) and a portal imaging device 1450. The gantry 1410 may be rotated to an angle corresponding to a selected projection and used to acquire an x-ray image of a VOI of a patient 1430 on a treatment couch 1440. The radiation source 1420 may then generate an x-ray beam that passes through the VOI of the patient 1430 and are incident on the portal imaging device 1450, creating an x-ray image of the VOI. After the x-ray image of the VOI has been generated, the beam energy of the radiation source 1420 may be increased so the radiation source 1420 may generate a treatment beam to treat a target region of the patient 1430. The present embodiment excludes therapeutic methods in which the movement of the patient is tracked and where a treatment step is performed after imagining of the VOI at the first angle but before imaging of the VOI at the second angle.

Alternatively, the kV imaging source and methods of operations described herein may be used with yet other types of gantry-based systems. In some gantry-based systems, the gantry rotates the kV imaging source and LINAC around an axis passing through the isocenter. Gantry-based systems include ring gantries having generally toroidal shapes in which the patient's body extends through the bore of the ring/toroid, and the kV imaging source and LINAC are mounted on the perimeter of the ring and rotates about the axis passing through the isocenter. Gantry-based systems may further include C-arm gantries, in which the kV imaging source and LINAC are mounted, in a cantilever-like manner, over and rotates about the axis passing through the isocenter. In another embodiment, the kV imaging source and LINAC may be used in a robotic arm-based system, which includes a robotic arm to which the kV imaging source and LINAC are mounted.

Unless stated otherwise as apparent from the foregoing discussion, it will be appreciated that terms such as "processing," "computing," "generating," "comparing" "determining," "calculating," "performing," "identifying," or the like may refer to the actions and processes of a computer system, or similar electronic computing device, that manipulates and transforms data represented as physical (e.g., electronic) quantities within the computer system's registers and memories into other data similarly represented as physical within the computer system memories or registers or other such information storage or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, embodiments of the present disclosure are not described with reference to any particular programming language. It will be appreciated that a variety of programming languages may be used to implement embodiments of the present disclosure.

It should be noted that the methods and apparatus described herein are not limited to use only with medical diagnostic imaging and treatment. In alternative embodiments, the methods and apparatus herein may be used in applications outside of the medical technology field, such as industrial imaging and non-destructive testing of materials. In such applications, for example, "treatment" may refer generally to the effectuation of an operation controlled by the treatment planning system, such as the application of a beam (e.g., radiation, acoustic, etc.) and "target" may refer to a non-anatomical object or area.

The above description of illustrated embodiments of the disclosure, including what is described in the Abstract, is not intended to be exhaustive or to limit the disclosure to the precise forms disclosed. While specific embodiments of, and examples for, the disclosure are described herein for illustrative purposes, various equivalent modifications are possible within the scope of the disclosure, as those skilled in the relevant art will recognize. The words "example" or "exemplary" are used herein to mean serving as an example, instance, or illustration. Any aspect or design described herein as "example" or "exemplary" is not necessarily to be construed as preferred or advantageous over other aspects or designs. Rather, use of the words "example" or "exemplary" is intended to present concepts in a concrete fashion. As used in this application, the term "or" is intended to mean an inclusive "or" rather than an exclusive "or". That is, unless specified otherwise, or clear from context, "X includes A or B" is intended to mean any of the natural inclusive permutations. That is, if X includes A; X includes B; or X includes both A and B, then "X includes A or B" is satisfied under any of the foregoing instances. In addition, the articles "a" and "an" as used in this application and the appended claims should generally be construed to mean "one or more" unless specified otherwise or clear from context to be directed to a singular form. Moreover, use of the term "an embodiment" or "one embodiment" or "an implementation" or "one implementation" throughout is not intended to mean the same embodiment or implementation unless described as such. Furthermore, the terms "first," "second," "third," "fourth," etc. as used herein are meant as labels to distinguish among different elements and may not necessarily have an ordinal meaning according to their numerical designation.

In the foregoing specification, the disclosure has been described with reference to specific exemplary embodiments thereof. It will, however, be evident that various modifications and changes may be made thereto without departing from the broader scope of the disclosure as set forth in the appended claims. The specification and drawings are, accordingly, to be regarded in an illustrative sense rather than a restrictive sense.

## Claims

1. A non-therapeutic method comprising:
imaging with an x-ray beam (910) having a first field of view (FOV) (1020) of a volume of interest (VOI) comprising a region of interest (ROI) of a patient (130) from a first position with an imager;
imaging the first FOV (1020) of the VOI comprising the ROI of the patient (130) from a second position with the imager;
receiving a first identification of a first portion of the imaged VOI from the first position designating the ROI to be imaged;
receiving a second identification of a second portion of the imaged VOI from the second position designating the ROI to be imaged;
in response to receiving the first identification, adjusting a variable aperture (750) of a collimator (160) of an imaging source (150) adapted to produce an imaging x-ray beam (910) to a second FOV (1050) corresponding to the ROI from the first position, the second FOV (1050) being different than the first FOV (1020) from the first position;
imaging the ROI of the patient (130) from the first position using the second FOV (1050);
in response to receiving the second identification, adjusting the aperture (750) of the collimator (160) of the imaging source (150) to a third FOV (1070) corresponding to the ROI from the second position, the third FOV (1070) being different than the first FOV (1020) from the second position; and
imaging the ROI of the patient (130) from the second position using the third FOV (1070).

2. A system comprising:
an x-ray imager comprising an x-ray imaging source (150) and an x-ray detector (170), wherein the x-ray imaging source (150) comprises a variable aperture collimator (160) adapted to produce an imaging x-ray beam (910);
a processing device, operatively coupled to the x-ray imager, to:
image with an x-ray beam (910) having a first field of view (FOV) (1020) of a volume of interest (VOI) comprising a region of interest (ROI) of a patient (130) from a first position with the x-ray imager;
image the first FOV (1020) of the VOI comprising the ROI of the patient from a second position with the x-ray imager;
receive a first identification of a first portion of the imaged VOI from the first position designating the ROI to be imaged;
receive a second identification of a second portion of the imaged VOI from the second position designating the ROI to be imaged;
in response to receiving the first identification, adjust the variable aperture (750) of the x-ray imaging source (150) to a second FOV (1050) corresponding to the ROI from the first position, the second FOV (1050) being different than the first FOV (1020) from the first position;
image the ROI of the patient (130) from the first position using the second FOV (1050);
in response to receiving the second identification, adjust the variable aperture (750) of the x-ray imaging source (150) to a third FOV (1070) corresponding to the ROI from the second position, the third FOV (1070) being different than the first FOV (1020) from the second position; and
image the ROI of the patient from the second position using the third FOV (1070).

3. The method of claim 1 or the system of claim 2, wherein the first identification of the first portion of the imaged VOI and the second identification of the second portion of the imaged VOI are based on a user selection of the ROI.

4. The method of claim 1 or the system of claim 2, wherein the first identification of the first portion of the imaged VOI and the second identification of the second portion of the imaged VOI are based on an automatic identification of the ROI.

5. The method of claim 1 or the system of claim 2, wherein the ROI in the VOI of the patient (130) comprises at least one fiducial marker (620) located within the VOI.

6. The method of claim 1 or the system of claim 2, wherein the ROI in the VOI of the patient comprises an anatomical feature located within the VOI.

7. The method of claim 1, wherein adjusting the aperture (750) of the imaging source (150) comprises adjusting a size of the aperture (750) to correspond to a size of the ROI.

8. The method of claim 1, wherein adjusting the aperture (750) of the imaging source (150) comprises adjusting a shape of the aperture (750) or wherein the aperture (750) of the imaging source (150) is adjusted while the kV imager moves from the first angle to the second angle.

9. A non-transitory computer-readable storage medium having instructions that, when executed by a processing device, cause the processing device to:
image, with an x-ray beam (910) having a first field of view (FOV) (1020) of a volume of interest (VOI)comprising a region of interest (ROI) of a patient (130) from a first position with an x-ray imaging source (150) comprising a variable aperture collimator (160) adapted to produce an imaging x-ray beam (910);
image the first FOV (1020) of the VOI comprising the ROI of the patient (130) from a second position with the x-ray imager;
receive a first identification of a first portion of the imaged VOI from the first position designating the ROI to be imaged;
receive a second identification of a second portion of the imaged VOI from the second position designating the ROI to be imaged;
in response to receiving the first identification, adjust a variable aperture collimator (160) of the x-ray imaging source (150) to a second FOV (1050) corresponding to the ROI from the first position, the second FOV (1050) being different than the first FOV (1020) from the first position;
image, from a second position with the x-ray imager, the VOI using the second FOV (1050);
in response to receiving the second identification, adjust the variable aperture (750) of the x-ray imaging source (150) to a third FOV (1070) corresponding to the ROI from the second position, the third FOV (1070) being different than the first FOV (1020) from the second position; and
image the ROI of the patient from the second position using the third FOV (1070).

10. The system of claim 2 or the non-transitory computer-readable storage medium of claim 9, wherein adjusting the aperture (750) of the x-ray imaging source (150) comprises adjusting a size of the aperture (750) to correspond to a size of the ROI.

11. The system of claim 2, wherein adjusting the aperture (750) of the x-ray imaging source (150) comprises adjusting a shape of the aperture (750).

12. The system of claim 2 or the non-transitory computer-readable storage medium of claim 9, wherein the aperture (750) of the x-ray imaging source (150) is adjusted while the imager moves from the first angle to the second angle.

13. The system of claim 2, wherein the x-ray imaging source (150) comprises a kilovoltage (kV) x-ray imaging source or wherein the x-ray imaging source (150) comprises a megavoltage (MV) x-ray imaging source.

14. The non-transitory computer-readable storage medium of claim 9, wherein adjusting the aperture (750) of the x-ray imaging source (150) comprises adjusting a shape of the aperture (750) to correspond to a shape of the ROI.

## Patentansprüche

1. Nicht-therapeutisches Verfahren, das Folgendes beinhaltet:
Abbilden, mit einem Röntgenstrahl (910) mit einem ersten Sichtfeld (FOV) (1020), eines Volumens von Interesse (VOI), das eine Region von Interesse (ROI) eines Patienten (130) umfasst, aus einer ersten Position mit einem Abbildungsgerät;
Abbilden des ersten FOV (1020) des VOI, das die ROI des Patienten (130) umfasst, aus einer zweiten Position mit dem Abbildungsgerät;
Empfangen einer ersten Identifikation eines ersten Teils des abgebildeten VOI aus der ersten Position, die die abzubildende ROI bezeichnet;
Empfangen einer zweiten Identifikation eines zweiten Teils des abgebildeten VOI aus der zweiten Position, die die abzubildende ROI bezeichnet;
Einstellen, als Reaktion auf den Empfang der ersten Identifikation, einer variablen Apertur (750) eines Kollimators (160) einer Abbildungsquelle (150), die zum Produzieren eines Abbildungsröntgenstrahls (910) auf ein zweites FOV (1050) entsprechend der ROI aus der ersten Position ausgelegt ist, wobei sich das zweite FOV (1050) vom ersten FOV (1020) aus der ersten Position unterscheidet;
Abbilden der ROI des Patienten (130) aus der ersten Position unter Verwendung des zweiten FOV (1050);
Einstellen, als Reaktion auf den Empfang der zweiten Identifikation, der Apertur (750) des Kollimators (160) der Abbildungsquelle (150) auf ein drittes FOV (1070) entsprechend der ROI aus der zweiten Position, wobei sich das dritte FOV (1070) vom ersten FOV (1020) aus der zweiten Position unterscheidet; und
Abbilden der ROI des Patienten (130) aus der zweiten Position unter Verwendung des dritten FOV (1070).

2. System, das Folgendes umfasst:
ein Röntgenabbildungsgerät mit einer Röntgenabbildungsquelle (150) und einem Röntgendetektor (170), wobei die Röntgenabbildungsquelle (150) einen Kollimator (160) mit variabler Apertur umfasst, der zum Erzeugen eines Abbildungsröntgenstrahls (910) geeignet ist;
ein Verarbeitungsgerät, mit dem Röntgenabbildungsgerät operativ gekoppelt zum:
Abbilden, mit einem Röntgenstrahl (910) mit einem ersten Sichtfeld (FOV) (1020), eines Volumens von Interesse (VOI), das eine Region von Interesse (ROI) eines Patienten (130) umfasst, aus einer ersten Position mit dem Röntgenabbildungsgerät;
Abbilden des ersten FOV (1020) des VOI, das die ROI des Patienten (130) umfasst, aus einer zweiten Position mit dem Röntgenabbildungsgerät;
Empfangen einer ersten Identifikation eines ersten Teils des abgebildeten VOI aus der ersten Position, die die abzubildende ROI bezeichnet;
Empfangen einer zweiten Identifikation eines zweiten Teils des abgebildeten VOI aus der zweiten Position, die die abzubildende ROI bezeichnet;
Einstellen, als Reaktion auf den Empfang der ersten Identifikation, der variablen Apertur (750) der Röntgenabbildungsquelle (150) auf ein zweites FOV (1050) entsprechend der ROI aus der ersten Position, wobei sich das zweite FOV (1050) vom ersten FOV (1020) aus der ersten Position unterscheidet;
Abbilden der ROI des Patienten (130) aus der ersten Position unter Verwendung des zweiten FOV (1050);
Einstellen, als Reaktion auf den Empfang der zweiten Identifikation, der variablen Apertur (750) der Röntgenabbildungsquelle (150) auf ein drittes FOV (1070) entsprechend der ROI aus der zweiten Position, wobei sich das dritte FOV (1070) vom ersten FOV (1020) aus der zweiten Position unterscheidet; und
Abbilden der ROI des Patienten aus der zweiten Position unter Verwendung des dritten FOV (1070).

3. Verfahren nach Anspruch 1 oder System nach Anspruch 2, wobei die erste Identifikation des ersten Teils des abgebildeten VOI und die zweite Identifikation des zweiten Teils des abgebildeten VOI auf einer Benutzerauswahl der ROI basieren.

4. Verfahren nach Anspruch 1 oder System nach Anspruch 2, wobei die erste Identifikation des ersten Teils des abgebildeten VOI und die zweite Identifikation des zweiten Teils des abgebildeten VOI auf einer automatischen Identifikation der ROI basieren.

5. Verfahren nach Anspruch 1 oder System nach Anspruch 2, wobei die ROI im VOI des Patienten (130) mindestens eine innerhalb des VOI befindliche Passermarke (620) umfasst.

6. Verfahren nach Anspruch 1 oder System nach Anspruch 2, wobei die ROI im VOI des Patienten ein innerhalb des VOI befindliches anatomisches Merkmal umfasst.

7. Verfahren nach Anspruch 1, wobei das Einstellen der Apertur (750) der Abbildungsquelle (150) das Einstellen einer Größe der Apertur (750) entsprechend einer Größe der ROI umfasst.

8. Verfahren nach Anspruch 1, wobei das Einstellen der Apertur (750) der Abbildungsquelle (150) das Einstellen einer Form der Apertur (750) beinhaltet oder wobei die Apertur (750) der Abbildungsquelle (150) eingestellt wird, während sich das kV-Abbildungsgerät vom ersten Winkel zum zweiten Winkel bewegt.

9. Nichtflüchtiges computerlesbares Speichermedium mit Befehlen, die bei Ausführung durch ein Verarbeitungsgerät das Verarbeitungsgerät veranlassen zum:
Abbilden, mit einem Röntgenstrahl (910) mit einem ersten Sichtfeld (FOV) (1020), eines Volumens von Interesse (VOI), das eine Region von Interesse (ROI) eines Patienten (130) umfasst, aus einer ersten Position mit einer Röntgenabbildungsquelle (150), die einen Kollimator (160) mit variabler Apertur umfasst, der zum Erzeugen eines Abbildungsröntgenstrahls (910) ausgelegt ist;
Abbilden des ersten FOV (1020) des VOI, das die ROI des Patienten (130) umfasst, aus einer zweiten Position mit dem Röntgenabbildungsgerät;
Empfangen einer ersten Identifikation eines ersten Teils des abgebildeten VOI aus der ersten Position, die die abzubildende ROI bezeichnet;
Empfangen einer zweiten Identifikation eines zweiten Teils des abgebildeten VOI aus der zweiten Position, die die abzubildende ROI bezeichnet;
Einstellen, als Reaktion auf den Empfang der ersten Identifikation, eines Kollimators (160) mit variabler Apertur der Röntgenabbildungsquelle (150) auf ein zweites FOV (1050) entsprechend der ROI aus der ersten Position, wobei sich das zweite FOV (1050) vom ersten FOV (1020) aus der ersten Position unterscheidet;
Abbilden, aus einer zweiten Position mit dem Röntgenabbildungsgerät, des VOI unter Verwendung des zweiten FOV (1050);
Einstellen, als Reaktion auf den Empfang der zweiten Identifikation, der variablen Apertur (750) der Röntgenabbildungsquelle (150) auf ein drittes FOV (1070) entsprechend der ROI aus der zweiten Position, wobei sich das dritte FOV (1070) vom ersten FOV (1020) aus der zweiten Position unterscheidet; und
Abbilden der ROI des Patienten aus der zweiten Position unter Verwendung des dritten FOV (1070).

10. System nach Anspruch 2 oder nichtflüchtiges computerlesbares Speichermedium nach Anspruch 9, wobei das Einstellen der Apertur (750) der Röntgenabbildungsquelle (150) das Einstellen einer Größe der Apertur (750) entsprechend einer Größe der ROI beinhaltet.

11. System nach Anspruch 2, wobei das Einstellen der Apertur (750) der Röntgenabbildungsquelle (150) das Einstellen einer Form der Apertur (750) beinhaltet.

12. System nach Anspruch 2 oder nichtflüchtiges computerlesbares Speichermedium nach Anspruch 9, wobei die Apertur (750) der Röntgenabbildungsquelle (150) eingestellt wird, während sich das Abbildungsgerät vom ersten Winkel zum zweiten Winkel bewegt.

13. System nach Anspruch 2, wobei die Röntgenabbildungsquelle (150) eine Kilospannung-(kV)-Röntgenabbildungsquelle umfasst oder wobei die Röntgenabbildungsquelle (150) eine Megaspannung-(MV)-Röntgenabbildungsquelle umfasst.

14. Nichtflüchtiges computerlesbares Speichermedium nach Anspruch 9, wobei das Einstellen der Apertur (750) der Röntgenabbildungsquelle (150) das Einstellen einer Form der Apertur (750) entsprechend einer Form der ROI beinhaltet.

## Revendications

1. Un procédé non thérapeutique comprenant :
l'imagerie avec un faisceau de rayons X (910) possédant un premier champ de vision (FOV) (1020) d'un volume d'intérêt (VOI) comprenant une région d'intérêt (ROI) d'un patient (130) à partir d'une première position avec un imageur,
l'imagerie du premier FOV (1020) du VOI comprenant la ROI du patient (130) à partir d'une deuxième position avec l'imageur,
la réception d'une première identification d'une première partie du VOI imagé à partir de la première position désignant la ROI à imager,
la réception d'une deuxième identification d'une deuxième partie du VOI imagé à partir de la deuxième position désignant la ROI à imager,
en réponse à la réception de la première identification, l'ajustement d'une ouverture variable (750) d'un collimateur (160) d'une source d'imagerie (150) adapté de façon à produire un faisceau de rayons X d'imagerie (910) sur un deuxième FOV (1050) correspondant à la ROI à partir de la première position, le deuxième FOV (1050) étant différent du premier FOV (1020) à partir de la première position,
l'imagerie de la ROI du patient (130) à partir de la première position au moyen du deuxième FOV (1050),
en réponse à la réception de la deuxième identification, l'ajustement de l'ouverture (750) du collimateur (160) de la source d'imagerie (150) sur un troisième FOV (1070) correspondant à la ROI à partir de la deuxième position, le troisième FOV (1070) étant différent du premier FOV (1020) à partir de la deuxième position, et
l'imagerie de la ROI du patient (130) à partir de la deuxième position au moyen du troisième FOV (1070).

2. Un système comprenant :
un imageur à rayons X comprenant une source d'imagerie à rayons X (150) et un détecteur à rayons X (170), où la source d'imagerie à rayons X (150) comprend un collimateur à ouverture variable (160) adapté de façon à produire un faisceau de rayons X d'imagerie (910),
un dispositif de traitement, couplé de manière opérationnelle à l'imageur à rayons X, de façon à :
imager avec un faisceau de rayons X (910) possédant un premier champ de vision (FOV) (1020) d'un volume d'intérêt (VOI) comprenant une région d'intérêt (ROI) d'un patient (130) à partir d'une première position avec l'imageur à rayons X,
imager le premier FOV (1020) du VOI comprenant la ROI du patient à partir d'une deuxième position avec l'imageur à rayons X,
recevoir une première identification d'une première partie du VOI imagé à partir de la première position désignant la ROI à imager,
recevoir une deuxième identification d'une deuxième partie du VOI imagé à partir de la deuxième position désignant la ROI à imager,
en réponse à la réception de la première identification, ajuster l'ouverture variable (750) de la source d'imagerie à rayons X (150) sur une deuxième FOV (1050) correspondant à la ROI à partir de la première position, le deuxième FOV (1050) étant différent du premier FOV (1020) à partir de la première position,
imager la ROI du patient (130) à partir de la première position au moyen du deuxième FOV (1050),
en réponse à la réception de la deuxième identification, ajuster l'ouverture variable (750) de la source d'imagerie à rayons X (150) sur une troisième FOV (1070) correspondant à la ROI à partir de la deuxième position, le troisième FOV (1070) étant différent du premier FOV (1020) à partir de la deuxième position, et
imager la ROI du patient à partir de la deuxième position au moyen du troisième FOV (1070).

3. Le procédé selon la Revendication 1 ou le système selon la Revendication 2, où la première identification de la première partie du VOI imagé et la deuxième identification de la deuxième partie du VOI imagé sont basées sur une sélection par l'utilisateur de la ROI.

4. Le procédé selon la Revendication 1 ou le système selon la Revendication 2, où la première identification de la première partie du VOI imagé et la deuxième identification de la deuxième partie du VOI imagé sont basées sur une identification automatique de la ROI.

5. Le procédé selon la Revendication 1 ou le système selon la Revendication 2, où la ROI dans le VOI du patient (130) comprend au moins un marqueur de centrage (620) situé à l'intérieur du VOI.

6. Le procédé selon la Revendication 1 ou le système selon la Revendication 2, où la ROI dans le VOI du patient comprend une caractéristique anatomique située à l'intérieur du VOI.

7. Le procédé selon la Revendication 1, où l'ajustement de l'ouverture (750) de la source d'imagerie (150) comprend l'ajustement d'une taille de l'ouverture (750) de façon à correspondre à une taille de la ROI.

8. Le procédé selon la Revendication 1, où l'ajustement de l'ouverture (750) de la source d'imagerie (150) comprend l'ajustement d'une forme de l'ouverture (750) ou où l'ouverture (750) de la source d'imagerie (150) est ajustée pendant que l'imageur kV se déplace du premier angle vers le deuxième angle.

9. Un support à mémoire lisible par ordinateur non transitoire possédant des instructions qui, lorsqu'elles sont exécutées par un dispositif de traitement, amènent le dispositif de traitement à :
imager, avec un faisceau de rayons X (910) possédant un premier champ de vision (FOV) (1020) d'un volume d'intérêt (VOI) comprenant une région d'intérêt (ROI) d'un patient (130) à partir d'une première position avec une source d'imagerie à rayons X (150) comprenant un collimateur à ouverture variable (160) adapté de façon à produire un faisceau de rayons X d'imagerie (910),
imager le premier FOV (1020) du VOI comprenant la ROI du patient (130) à partir d'une deuxième position avec l'imageur à rayons X,
recevoir une première identification d'une première partie du VOI imagé à partir de la première position désignant la ROI à imager,
recevoir une deuxième identification d'une deuxième partie du VOI imagé à partir de la deuxième position désignant la ROI à imager,
en réponse à la réception de la première identification, ajuster un collimateur à ouverture variable (160) de la source d'imagerie à rayons X (150) sur une deuxième FOV (1050) correspondant à la ROI à partir de la première position, le deuxième FOV (1050) étant différent du premier FOV (1020) à partir de la première position,
imager, à partir d'une deuxième position avec l'imageur à rayons X, le VOI au moyen du deuxième FOV (1050),
en réponse à la réception de la deuxième identification, ajuster l'ouverture variable (750) de la source d'imagerie à rayons X (150) sur un troisième FOV (1070) correspondant à la ROI à partir de la deuxième position, le troisième FOV (1070) étant différent du premier FOV (1020) à partir de la deuxième position, et
imager la ROI du patient à partir de la deuxième position au moyen du troisième FOV (1070).

10. Le système selon la Revendication 2 ou le support à mémoire lisible par ordinateur non transitoire selon la Revendication 9, où l'ajustement de l'ouverture (750) de la source d'imagerie à rayons X (150) comprend l'ajustement d'une taille de l'ouverture (750) de façon à correspondre à une taille de la ROI.

11. Le système selon la Revendication 2, où l'ajustement de l'ouverture (750) de la source d'imagerie à rayons X (150) comprend l'ajustement d'une forme de l'ouverture (750).

12. Le système selon la Revendication 2 ou le support à mémoire lisible par ordinateur non transitoire selon la Revendication 9, où l'ouverture (750) de la source d'imagerie à rayons X (150) est ajustée pendant que l'imageur se déplace du premier angle vers le deuxième angle.

13. Le système selon la Revendication 2, où la source d'imagerie à rayons X (150) comprend une source d'imagerie à rayons X kilovoltage (kV) ou où la source d'imagerie à rayons X (150) comprend une source d'imagerie à rayons X mégavoltage (MV).

14. Le support à mémoire lisible par ordinateur non transitoire selon la Revendication 9, où l'ajustement de l'ouverture (750) de la source d'imagerie à rayons X (150) comprend l'ajustement d'une forme de l'ouverture (750) de façon à correspondre à une forme de la ROI.
